(19) **Europäisches Patentamt**
**European Patent Office**
**Office européen des brevets**

(11) **EP 4 679 363 A1**

(12) **EUROPEAN PATENT APPLICATION**

(43) Date of publication:
**14.01.2026 Bulletin 2026/03**

(21) Application number: **24306171.0**

(22) Date of filing: **11.07.2024**

(51) International Patent Classification (IPC):
*G06T 7/00* (2017.01)   *G06T 7/11* (2017.01)

(52) Cooperative Patent Classification (CPC):
**G06T 7/0012; G06T 7/11;** G06T 2207/10092;
G06T 2207/30016

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Dassault Systèmes**
**78140 Vélizy-Villacoublay (FR)**

(72) Inventors:
• **Kouayi, Emilie**
  **Vélizy-Villacoublay (FR)**
• **Moreau, Louise**
  **Vélizy-Villacoublay (FR)**

(74) Representative: **Bandpay & Greuter**
**11 rue Christophe Colomb**
**75008 Paris (FR)**

(54) **PARCELING GREY MATTER OF A BRAIN OF A HUMAN PATIENT**

(57) The disclosure notably relates to a computer-implemented method for parceling grey matter of a human brain of a human patient comprising obtaining a tractogram including tractogram streamlines, each having a first extremity located in a first portion of grey matter of a second extremity located in a second portion of grey matter. The parceling method also comprises using a predetermined clustering algorithm to obtain tractogram streamline clusters, and, for each cluster of at least a part of the clusters, identifying a respective first region of grey matter including for each streamline of the cluster its first extremity, and a respective second region of grey matter including for each streamline of the cluster its second extremity. The parceling method also comprises determining a parcellation based on the identified regions, including an iterative merging process which includes merging pairs of regions based on a metric quantifying an overlap.

**Description**

**TECHNICAL FIELD**

The disclosure relates to the field of computer programs and systems, and more specifically to a method, system and program for parceling grey matter of a brain of a human patient.

**BACKGROUND**

The brain is made of the white matter, which consists of fibers that conduct the electrical pulses, and of the grey matter located deeply within the brain and at the edges of the brain, which receives the electrical pulses and treats them. The modelling of the grey matter of the brain may be used for assisting clinicians to perform diagnostics around a common representation of contextualized grey matter data in order to take a decision about the diagnosis, treatment or monitoring of a pathology. The grey matter data may represent, e.g., anatomical information such as cortical and sub-cortical grey matter, or functional information such as the location of motor or vision areas.

Within this context, there is still a need for an improved solution for parceling grey matter of a brain of a human patient.

**SUMMARY**

It is therefore provided a computer-implemented method for parceling grey matter of a human brain of a human patient. The parceling method comprises obtaining a tractogram of the brain of the human patient. The tractogram includes tractogram streamlines. Each streamline has a first extremity located in a first portion of grey matter of the brain of the human patient, and a second extremity located in a second portion of grey matter of the brain of the human patient. The first portion and the second portion are separate. The parceling method also comprises using a predetermined clustering algorithm to obtain a plurality of tractogram streamline clusters. The parceling method also comprises, for each tractogram streamline cluster of at least a part of the plurality of tractogram streamline clusters, identifying a respective first region of grey matter. The respective first region of grey matter includes, for each tractogram streamline of the cluster, its first extremity. The parceling method also comprises identifying a respective second region of grey matter includes, for each tractogram streamline of the cluster, its second extremity. The respective first region is separate from the respective second region. The parceling method also comprises determining a parcellation based on the identified regions. The determining of the parcellation includes an iterative merging process. The iterative merging process includes, at each iteration, merging pairs of regions. Merging pairs of regions is based on a metric quantifying an overlap.
The method may comprise one or more of the following:

- the metric quantifying an overlap is a dice score;
- the iterative merging process includes a stage comprising, at each iteration:

  ○ for each pair of regions, determining a value of the metric, the value of the metric thereby quantifying an overlap between the pair of regions;
  ○ merging one or more respective pairs of regions each having a value of the metric above a first predetermined threshold, for example a pair of regions having a highest value of the metric among all pairs of regions;

  the stage of the iterative process being performed until no pair of regions having a value of the metric above the first predetermined threshold exists;
- the method further comprises:

  ○ prior to the iterative merging process, determining a graph comprising, for each tractogram streamline cluster, a first node representing the respective first region and a second node representing the respective second region, and an edge connecting the first node and the second node, the edge thereby representing a respective tractogram streamline cluster,
  ○ at each iteration of the stage, merging each pair of nodes of the graph representing a respective merged pair of regions; and
  ○ after the stage, evaluating whether the graph is connected, wherein if the graph is not connected, the iterative merging process includes a further stage comprising iteratively merging one or more pairs of regions and each pair of nodes of the graph representing a merged pair of regions;

- the further stage comprises:

  ○ identifying a principal connected component of the graph and one or more secondary connected components of the graph; and
  ○ performing, iteratively, an explorative process on pairs of a respective node of the principal connected component and a node of a respective secondary connected component, the explorative process performing at each iteration for a respective pair:

    ▪ re-determining a value of the metric quantifying an overlap between regions corresponding to the respective pair, and
    ▪ merging the node of the respective secondary component with the respective node of a principal connected component having the highest value of the metric quantifying an overlap between the corresponding regions, the explorative process being performed until there exists no secondary component having a node representing a region forming an overlap for which the value of the metric is above zero with a region represented by a node of the principal connected component;

- determining the parcellation comprises, after the iterative merging process, performing an iterative post-processing over remaining regions of grey matter, the iterative post-processing comprising, at each iteration:

    ○ determining pairs of overlapping regions, and determining a portion representing the overlap between the regions;
    ○ computing respective Mahalanobis distances between a respective region of the pair and the determined portion; and
    ○ assigning the portion to a respective region of the pair having the smallest Mahalanobis distance

- the respective Mahalanobis distance is of the type:

$$d_{Maha} = \left\| \frac{x - \mu_{cluster}}{\sigma_{cluster}} \right\|_2 ,$$

wherein $\mu_{cluster}$ is the mean of voxels of a corresponding region of the pair, $\sigma_{cluster}$ is the standard deviation of voxels of the corresponding region of the pair, and x is a position of a voxel belonging to the predetermined portion;

- the method further comprises, after performing the iterative post-processing:

    ○ determining small regions, each small region being a region having an average number of voxels below a second predetermined threshold ;
    ○ for each small region:

        ■ determining neighboring large regions, a large region being a region having an average number of voxels above the second predetermined threshold,
        ■ counting a most present anatomical category among anatomical categories associated to each respective determined neighboring large region;
        ■ assigning voxels of the small region to the respective neighboring large region having the largest number of an anatomical category;

- the predetermined clustering algorithm comprises for a respective plurality of tractogram streamlines:

    ○ obtaining (Q10) a threshold value as a third predetermined threshold;
    ○ assigning (Q20) an initial tractogram streamline to an initial tractogram streamline cluster;
    ○ iteratively visiting (Q30) subsequent tractogram streamlines of the respective plurality of tractogram streamlines; and
    ○ for each respective subsequent tractogram streamline and with respect to a predetermined distance (Q40):

        ■ computing (Q410) a respective distance value between the subsequent tractogram streamline and a centroid of each already-existing tractogram streamline cluster; and
        ■ determining (Q420) a respective tractogram streamline cluster having a smallest distance value:

            • if the respective distance value is below the third predetermined threshold (Q430), assigning (Q431) the respective subsequent tractogram streamline to the respective tractogram streamline cluster;
            • else, creating (Q432) a subsequent tractogram streamline cluster and assigning the respective subsequent tractogram streamline to said subsequent tractogram streamline cluster;

- the predetermined clustering algorithm further comprises, after assigning all tractogram streamlines of the plurality of tractogram streamlines:

    ○ re-computing the centroid of each tractogram streamline cluster using the predetermined centroid computation algorithm; and
    ○ for each tractogram streamline assigned to a respective tractogram streamline cluster, and with respect to the predetermined distance:

        ■ computing a respective distance value between the tractogram streamline and the centroid of the respective tractogram streamline cluster;
        ■ un-assigning the tractogram streamline from the respective tractogram streamline cluster if the respective distance value is above the third predetermined threshold;

    ○ re-computing again the centroid of each tractogram streamline cluster using the predetermined centroid computation algorithm; and
∘ for each tractogram streamline un-assigned to another respective tractogram streamline cluster, and with respect to the predetermined distance:

■ computing a respective distance value between the tractogram streamline and the centroid of each tractogram streamline cluster;
■ determining a tractogram streamline cluster having a smallest distance value:

• if the respective distance value is below the third predetermined threshold, re-assigning the unassigned tractogram streamline to the determined tractogram streamline cluster;
• else creating a subsequent tractogram streamline cluster and re-assigning the subsequent tractogram streamline to a subsequent tractogram streamline cluster;

- the predetermined distance is a minimum direct-flip distance; and
- the predetermined clustering algorithm obtains another threshold value, the other threshold value being higher than the threshold value, and prior to the steps of using the predetermined clustering algorithm, the method comprises:

∘ retrieving (P10) all the tractogram streamlines from the tractogram;
∘ applying (P20) the predetermined clustering algorithm to said all the tractogram streamlines to obtain an initial plurality of tractogram streamline clusters;
∘ selecting (P40) one or more initial sets of tractogram streamlines from the plurality of initial tractogram streamline clusters, a respective initial tractogram streamline cluster being selected as an initial set, the respective initial tractogram streamline cluster thereby fulfilling a coarser proximity criterion;

the using of the predetermined clustering algorithm to obtain the plurality of tractogram streamline clusters being applied to the streamlines of at least part of the one or more initial sets of tractogram streamlines.

It is further provided a computer program comprising instructions for performing the method.
It is further provided a computer readable storage medium having recorded thereon the computer program.
It is further provided a system comprising a processor coupled to a memory, the memory having recorded thereon the computer program.

**BRIEF DESCRIPTION OF THE DRAWINGS**

Non-limiting examples will now be described in reference to the accompanying drawings, where:

- FIG. 1 shows a flowchart of an example of the method;
- FIG. 2 shows an example of the system; and
- FIG.s 3 to 7 illustrate the method.

**DETAILED DESCRIPTION**

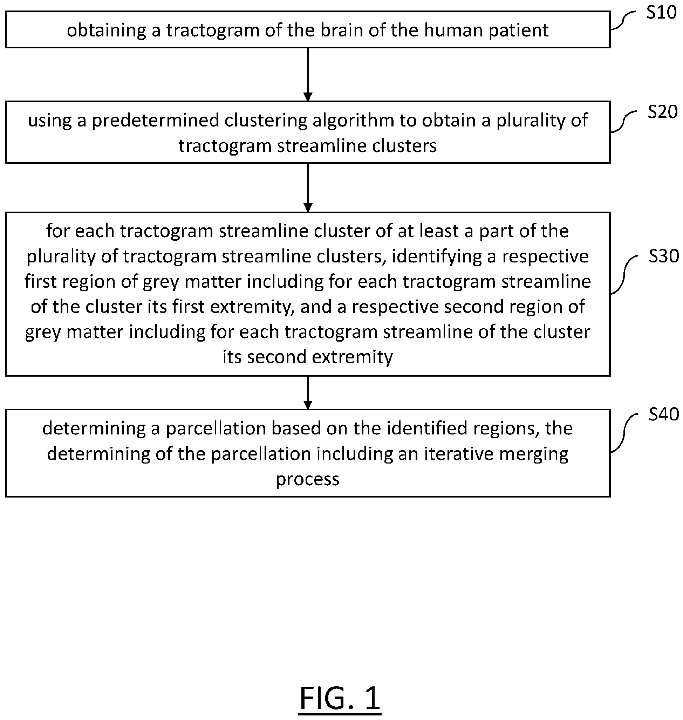

With reference to the flowchart of FIG. 1, it is proposed a computer-implemented method for parceling grey matter of a human brain of a human patient. The method comprises obtaining S10 a tractogram of the brain of the human patient. The tractogram includes tractogram streamlines. Each streamline has a first extremity located in a first portion of grey matter of the brain of the human patient, and a second extremity located in a second portion of grey matter of the brain of the human patient. The first portion and the second portion are separate one from another. The method also comprises using S20 a predetermined clustering algorithm to obtain a plurality of tractogram streamline clusters. The parceling method also comprises S30, for each tractogram streamline cluster of at least a part of the plurality of tractogram streamline clusters, identifying a respective first region of grey matter. The respective first region of grey matter includes, for each tractogram streamline of the cluster, its first extremity. The parceling method also comprises identifying a respective second region of grey matter. The respective second region of grey matter includes, for each tractogram streamline of the cluster, its second extremity. The respective first region is separate from the respective second region. The method also comprises determining S40 a parcellation based on the identified regions. The determining of the parcellation includes an iterative merging process. The iterative merging process includes, at each iteration, merging pairs of regions. Merging pairs of regions is based on a metric quantifying an overlap.
Such a method forms an improved solution for parceling grey matter of a brain of a human patient.
Notably, the method enables obtention of anatomical information of grey matter, based on the underlying connectivity found in the white matter fibrous structure of the brain that is represented by the tractogram streamlines. This enables an accurate parcellation.
More particularly, as the method initially identifies at S30 regions including extremities of tractogram streamlines and based on a clustering S20 of the tractogram streamlines, the method provides at S30 a first relevant parcellation of grey matter at the interface with white matter. Indeed, as the method uses the predetermined clustering algorithm to obtain the plurality of tractogram streamline clusters, the method groups tractogram streamlines having a similar connectivity profile, e.g., having a similar shape and/or spatial similarity between the respective extremities of the tractogram streamline clusters. Thus, when separate, the two extremal regions of each cluster (corresponding to the respective extremities of tractogram streamlines and represented white matter fibers) each represent a relevant region of grey matter at the interface with white matter, since they are connected one to another by white matter fibrous structure. By identifying at S30 for each such cluster a respective first region corresponding to one cluster extremity and a respective second region corresponding to the other cluster extremity, the method already reaches at S30 a parcellation which is biologically meaningful (in terms of underlying connectivity).

Now, the identifying S30 may provide a high number of regions, due to the clustering S20 reaching a high number of clusters. The parcellation obtained at S30 may thus be too granular, wherein coherent parcels of grey matter are (undesirably) subdivided in several regions, with no true biological separation therebetween. Thus, to determine the parcellation at S40 the method iteratively merges pairs of regions that overlap each other, based on metric quantifying the overlap. This enables the method to identify overlapping regions that biologically belong to a single parcel, and merge such regions together. This allows to reach an exploitable number of parcels in the final parcellation, and this further ensures that the parcellation is biologically accurate (i.e., regions/parcels of grey matter that are coherent based on connectivity - and thus in terms of brain activity- each correspond to one, and only one, parcel in the final parcellation, with no such coherent region being superfluously subdivided).

The method may further comprise displaying (e.g., to one or more medical practitioners) a 3D representation of the parcellation, for example showing/highlighting the grey matter represented by the regions, e.g., in a different color.

The parcellation based on the identified regions may be usable for diagnosing and/or treating a patient with respect to a medical condition. In other words, the method may comprise using the result of the method to perform a diagnosis step and/or a treatment step of which at least one parameter depends on said parcellation. For example, the method may comprise displaying (e.g., to one or more medical practitioners) a 3D representation of the parcellation, for example showing/highlighting the grey matter represented by the regions, e.g., in a different color. Alternatively or additionally, the output of S20 may be fed to an automatic process to perform such use.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

A typical example of computer-implementation of a method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g., one for the program, and possibly one for the database).

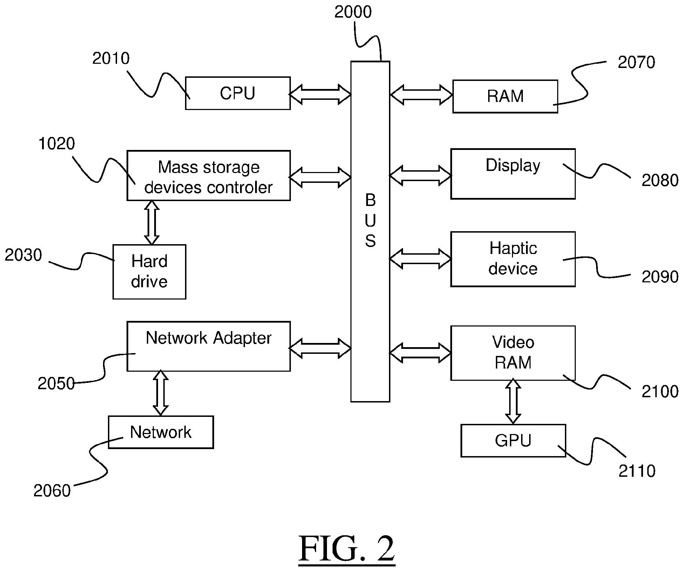

FIG. 2 shows an example of the system, wherein the system is a client computer system, e.g., a workstation of a user.

The client computer of the example comprises a central processing unit (CPU) 2010 connected to an internal communication BUS 2000, a random access memory (RAM) 2070 also connected to the BUS. The client computer is further provided with a graphical processing unit (GPU) 2110 which is associated with a video random access memory 2100 connected to the BUS. Video RAM 2100 is also known in the art as frame buffer. A mass storage device controller 2020 manages accesses to a mass memory device, such as hard drive 2030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 2050 manages accesses to a network 2060. The client computer may also include a haptic device 2090 such as cursor control device, a keyboard or the like. A cursor control device is used in the client computer to permit the user to selectively position a cursor at any desired location on display 2080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

The method is for parceling grey matter of a brain of a human patient. In other words, the method is for determining regions or "parcels" (e.g., cortical and/or sub-cortical) of grey matter of the brain represented by the tractogram. This means that the method takes as input a tractogram of the brain of the human patient and outputs (e.g., a 3D representation of) regions representing the grey matter. Each region or parcel is a coherent region in terms of brain function. The method thus allows to measure coherent regions of grey matter.

The method comprises obtaining at S10 a tractogram of the brain of the human patient. The tractogram includes tractogram streamlines. The tractogram streamlines may be represented in a 3D space.

The 3D space may be voxelized, meaning that the 3D space comprises a grid of voxels, for example covering the patient's brain. The tractogram streamlines may be represented in the voxel grid. The method may comprise computing the voxel grid, and/or computing an intersection between each streamline and the voxel grid, and/or between each streamline extremity and the voxel grid. The method may comprise determining for each first/second extremity identified at S30 the respective voxel containing the first/second extremity.

Any voxel described herein may be a volumetric element (e.g., a cube) which may be identified by three dimensional coordinates (e.g., x-y-z coordinates) corresponding to the location of the voxel in the voxel grid. The tractogram may be 3D represented into voxels via a projection into the voxel grid. In other words, the 3D representation into voxels may correspond to the intersection of the tractogram with voxels of a corresponding voxel grid. It is to be understood that steps of the method described herein may be performed on the voxel grid in any manner. The voxel grid may comprise more than 100 or 200 voxels in its width x, more than 100 or 200 voxels in its depth y, and/or more than 100 or 200 voxels in its height z, for example a 256x256x256 voxel grid. The voxel grid may be a cube. Each voxel may be a parallelepiped such as a cube, and represent a portion of the brain of width larger than 0.1 mm or 0.5mm and/or lower than 2mm or 1.5mm, of depth larger than 0.1 mm or 0.5mm and/or lower than 2mm or 1.5mm, and/or of height larger than 0.1 mm or 0.5mm and/or lower than 2mm or 1.5mm, for example a 1mm x 1mm x 1mm portion of the brain.

Each voxel may further include information such as one or more labels, each label having information of a respective anatomical category. For example a voxel intersecting with an extremity of a streamline may comprise a label indicative of grey matter.

Each first region identified at S30 may consist of the set of voxels each including a respective first extremity, and each second region identified at S30 may consist of the set of voxels each including a respective second extremity. A "region" herein may thereby refer to a set of voxels.

The tractogram streamlines may be constructed from a diffusion voxel model determined from a diffusion Magnetic Resonance Image (MRI).

Obtaining S10 the tractogram may comprise acquiring (i.e., physically measuring) and/or obtaining a diffusion MRI of the brain. The diffusion MRI may be obtained from physical measurements of the diffusion of water molecules within the brain performed via a diffusion magnetic resonance imaging process. White matter is a structure within the brain which is composed of fibers that gather into bundles. Thus, the water molecules present in the white matter spread within a constrained environment. Hence, the measurement of the diffusion of water molecule within the brain corresponds to finding the structure of the white matter.

Obtaining S10 the tractogram may also comprise determining the diffusion voxel model from the diffusion MRI. The diffusion voxel model may be a set of voxels represented in a voxel grid, wherein each voxel comprises indication of the diffusion of water molecules within the brain. In other words the diffusion voxel model is a 3D representation of the tractogram into voxels. The diffusion voxel model may be modeled from a mathematical model that determines the diffusion of water molecules from the diffusion MRI on each voxel.

The diffusion voxel model may be obtained from a Fiber Orientation Density Function (FOD), which provides a probability of the water molecule to spread into one preferential direction. Hence, a tractogram streamline represents the 3D line resulting from the computed probability that the water spreads into one preferential direction and thus how it moves along fibers of white-matter.

The obtaining S10 may also comprise constructing the tractogram from the diffusion voxel model. In other words, the obtaining S10 may gather the tractogram streamlines resulting from the diffusion voxel model which represent the computed probability that the water spreads into one preferential direction within the brain, as captured by the diffusion MRI. The construction of the tractogram may be performed by choosing an initial position in the diffusion MRI (also called seed position). The initial position may be chosen in an area of the diffusion MRI representing white matter. The construction may determine a main diffusion direction at the initial position using the diffusion voxel model. Constructing the tractogram streamlines may comprise, e.g., creating sequences of 3D points traversing the voxels of the diffusion voxel model, to follow this main diffusion direction until it reaches a stopping criterion or other voxel.

The tractogram obtained at S10 includes tractogram streamlines representing fibers of white-matter inside the brain and arriving at the interface with the grey matter. The tractogram may include full-fiber tractogram streamlines, i.e., tractogram streamlines that model the full length of fibers of white matter together with their interface with grey matter. The tractogram may include non-looping tractogram streamlines, i.e., representing fibers that do not return to a same portion of grey matter of the brain, thus ensuring connectivity between two separate portions of grey matter.

Each (full-fiber and non-looping) tractogram streamline has a first extremity located in a first portion of grey matter of the human patient. The tractogram streamline has a second extremity located in a second portion of grey matter of the human patient. For example, constructing the tractogram may comprise choosing placing a seed at a location of the diffusion MRI representing the interface between white and grey matter of the brain. The first extremity may correspond to the location of the seed. The second extremity may correspond to another position at the interface between white and grey matter of the brain. In other words, the stopping criterion may comprise reaching a position corresponding to the interface between white and grey matter of the brain.

The first portion and the second portion are separate for such a full-fiber and non-looping tractogram streamline. In other words, the first and second portion do not overlap on the tractogram (e.g., a concave hull of the first portion does not intersect a concave hull of the second portion, for example, the first and second portion share no voxel).

Depending on the technique used for acquiring the tractogram, the tractogram obtained at S10 may also include "sectioned-fiber" tractogram streamlines, i.e., representing sectional portions of fibers of white-matter wherein it is known that a tractogram acquisition may lead to such sectioned-fiber tractogram streamlines. Such sectional portions may have been cut erroneously during tractogram acquisition, and while possibly anatomically accurate along the sectional portion, these are incomplete. Indeed, the sectioned-fiber tractogram streamline exclude at least one extremity (e.g., both extremities of the sectioned-fiber tractogram streamline) located at respective portions of grey-matter. Optionally, an original tractogram including sectioned-fiber tractogram streamlines may be first acquired, and the method may comprise filtering out all sectioned-fiber tractogram streamlines, leaving only full-fiber tractogram streamlines at S10.

The method comprises using S20 a predetermined clustering algorithm to obtain a plurality of tractogram streamline clusters. The predetermined clustering algorithm may be represented by a set of instructions configured to take as input a respective plurality of tractogram streamlines, and to output a plurality of tractogram streamline clusters. At S20, the predetermined clustering algorithm may be applied with, as input, a plurality (e.g., a part of, or, all of) of the tractogram streamlines of the tractogram obtained at S10.

The predetermined clustering algorithm may assign tractogram streamlines (from the input tractogram) to a respective tractogram streamline cluster based on similarity and/or proximity criteria. The similarity and/or proximity criteria may comprise (e.g., one of or a combination of) criteria relative to the shape, geometrical similarity and/or localization of the tractogram streamlines. For example, obtaining a respective tractogram streamline cluster of the plurality may comprise assigning to the cluster tractogram streamlines having a similar shape and being close (in spatial proximity) one to another. Assigning tractogram streamlines to the respective tractogram streamline cluster may comprise associating the tractogram streamlines with data pieces (e.g., labels) indicative of the association with the tractogram cluster. The tractogram streamline cluster is thereby a set of tractogram streamlines satisfying the similarity criteria.

For each tractogram streamline cluster of at least a part of the plurality of tractogram streamline clusters (e.g. part of or all clusters having full-fiber and non-looping tractogram streamlines), the method comprises S30 identifying a respective first region of grey matter and a respective second region of grey matter. The respective first region includes for each (full-fiber and non-looping) tractogram streamline of the cluster its first extremity. The method also comprises identifying a respective second region of grey matter. The respective second region includes, for each tractogram streamline of the cluster its second extremity. In examples, each (first or second) extremity may be represented by a voxel located at the intersection of the extremity and the voxel grid. In other words, the method may identify at S30 voxels intersecting with the first and second extremities.

Identifying the respective first and second regions may comprise performing a k-means algorithm on the extremities of the tractogram streamlines of the cluster. For example, performing the k-means algorithm may comprise setting k=2 to identify the first and second regions. The first region may be identified as (an arbitrary) one among the two clusters of streamline extremities outputted by the k-means algorithm, and the second region may be identified as the other cluster outputted by the k-means algorithm.

Additionally or alternatively, the method may identify an extremity of a cluster comprising sectioned-fiber tractogram streamlines as being part of a respective first or second region (in case the method does not filter out existing sectioned-fiber tractogram streamlines).

For example, the method may comprise performing the clustering at S20 using as input all streamlines of the tractogram obtained at S10, even the sectioned-fiber ones, then identifying for each cluster, all streamline extremities that fall within the grey matter (thus excluding extremities of sectioned-fiber streamlines that do not fall within grey matter, in case the method has not filtered out sectioned-fiber streamlines; otherwise all extremities of full-fiber streamlines fall within grey matter). This allows to exclude extremities of sectioned-fiber tractogram streamlines that do not correspond to fiber extremities and thus do not fall within grey matter, and rather correspond to a point at an intermediate section of the fiber and thus fall inside white matter. The identification of streamline extremities that fall within the grey matter may comprise determining the voxel intersecting with each streamline extremity, and evaluating whether the voxel represents grey matter or not (e.g., whether the label associated with the voxel is indicative of grey matter).

The respective first region is separate from the respective second region, as a result of performing the k-means algorithm as explained above. The method may optionally exclude looping streamlines from the first or second region. For example, if the two obtained regions are too close one from another as a result of performing the k-means, e.g., when the distance between the two obtained regions is below a predetermined threshold, the method may consider that this a "looping" cluster and discard it.

The method comprises determining S40 the parcellation based on the identified regions. In other words, the method determines a first region and a second region of grey matter represented by the identified respective first region and respective second region. The determining of the parcellation includes an iterative merging process. The iterative merging process may comprise iterating on identified first regions and iterating on identified second regions.

The iterative merging process may include, at each iteration, merging pairs of regions based on a metric quantifying an overlap. In other words, the method iterates through pairs of regions and determines if a respective pair overlaps. By "merging" it is meant that the extremities included in each region of the pair are combined to form a single region. In other words, the single region comprises all the extremities included in the pair of regions. By "overlapping" it is meant that there exists an intersection between a concave hull of a region of the pair and another concave hull of a second region of the pair.

For example, any pair of regions described herein may overlap if there is at least one voxel common each region of the pair.

The iterative merging process may include a (first) stage comprising, at each iteration, for each pair of regions, determining a value of the metric. The value of the metric thereby quantifies an overlap between the pair of regions. The metric may take values in a non-negative range (e.g., of real numbers), i.e., greater than or equal than zero lower than a maximum number. The values of the metric may increase along the non-negative range as a measure of the relative overlap between the pair of regions. In other words, the values of the metric may increase if the overlap between the range of regions increases. For example, if the iterative merging process determines that, for a respective pair of regions, the value of the metric is zero, the pair of regions does not overlap. If the iterative merging process determines that, for the respective pair of regions, the value of the metric is the maximum number, the pair of regions completely overlaps.

The metric quantifying an overlap may be any measure of an extent of overlap between two regions, for example a dice score between the two regions. Let A denote a region of the pair and B denote the other region of the pair. The dice score may be defined as two times the number of extremities included in the region A and included in the region B over the sum of the number of extremities included in the region A and the number of extremities included in the region B.

The dice score may be of the type:

$$Dice\ Score = \frac{2 * A \cap B}{A + B}$$

If the dice score is close to one (e.g., approximatively equal to one), it means that the regions A and B have a large number of extremities in common, i.e., the two regions substantially overlap. If the dice score is close to zero (e.g., approximately equal to zero), it means that the regions A and B have few extremities in common, i.e., the two regions do not overlap substantially.

The first stage of the iterative merging process may comprise merging one or more respective (e.g., a plurality of) pairs of regions each having a value of the metric above a first predetermined threshold. The first predetermined threshold may be a non-negative number set in any manner. For example, when the metric is the dice score, the predetermined threshold may be any number in the range [0,1], e.g., 0.8. For example, the first stage may comprise merging a pair of regions having the highest value of the metric among all pairs of regions having the value of the metric above the predetermined threshold.

The first stage of the iterative merging process may be performed until no pair of regions having a value of the metric above the first predetermined threshold exists. In other words, the iterative process stops when determining that there are no pair of regions that significantly overlap.

An example of the first stage included in the iterative merging process is now discussed.

In a first iteration, the first stage may comprise selecting a pair regions, e.g., randomly (a pair of the identified first or second regions). The first stage may comprise determining a value of the dice score between the pair of regions. If the metric is below the first predetermined threshold, the first stage may comprise proceeding to select another pair of regions. Else, if the dice score is above the first predetermined threshold, the first stage may comprise merging the pair of regions having the highest value above the predetermined threshold, thereby creating a new region.

In a second iteration, the first stage may comprise selecting pairs of regions from the set of regions resulting from the first iteration (after the merging of the pair of regions in the first iteration). In the second iteration, the first stage may comprise determining a value of the dice score between respective pairs of regions. If the dice score is above the first predetermined threshold, in the second iteration, the first stage may comprise merging the pair of regions having the highest value above the predetermined threshold, thereby creating another new region.

The iterative merging process may continue to perform the first stage until achieving a stopping criterion. For example, the first stage may be performed until no pair of regions having a value of the dice score above the first predetermined threshold exists.

In an example, the iterative merging process may be followed by further post-processing as described below.

The method thus improves the parcellation of grey matter of the brain of the human patient. Indeed, the method exploits the connectivity of the tractogram streamlines to provide an accurate output. This is because the extremities of the tractogram streamlines are located at portions of gray matter. Hence, the parcellation follows the anatomical hypothesis that gray matter is connected by white matter fibers (represented by the tractogram streamlines). The method is efficient thanks to the use of the clustering algorithm. Indeed, the predetermined clustering algorithm assembles the tractogram streamlines having a similar shape and/or spatial location in respective clusters and thus reduces the dimensionality of the tractogram. A tractogram may contain millions of tractogram streamlines. The predetermined clustering algorithm allows the reduction of the number of tractogram streamlines and underlying connectivity paths. Hence, the method outputs the regions efficiently all while still providing an accurate result.

The method may further comprise, prior to the iterative merging process, determining a graph. Any graph *G* of this disclosure may be denoted as G = *(V, E),* where V is a set whose elements are called nodes (also called vertices), and E is a set of edges (also called links).

The graph comprises, for each tractogram streamline cluster, a first node representing the respective first region and a second node representing the respective second region. The graph also comprises an edge connecting the first node and the second node. The edge thereby represents a respective tractogram streamline cluster. In other words, the method comprises determining the graph from each tractogram streamline cluster and their respective region so that the graph comprises, for a respective tractogram streamline cluster, an edge representing the respective tractogram cluster connecting a pair of nodes representing the pair of the first region and second region of the respective tractogram cluster.

The method may further comprise, at each iteration of the first stage, merging each pair of nodes of the graph representing a respective merged pair of regions. In other words, the method comprises combining the pair of the nodes of the graph representing the merged pair of regions when determining that the pair of regions overlaps.

The method may further comprise, after the first stage (and while there are pair of regions having the value of the dice above the predetermined threshold, in other words while the iterative merging process continues), evaluating whether the graph is connected. In other words, the method comprises evaluating if for any pair of nodes, there is a path of edges linking the pair of nodes. If the graph is not connected, the iterative merging process may include a further (second) stage. In other words, the iterative merging process may include the first stage and the second stage at each iteration. The second stage may comprise iteratively merging one or more pairs of regions and each pair of nodes of the graph representing a merged pair of regions.

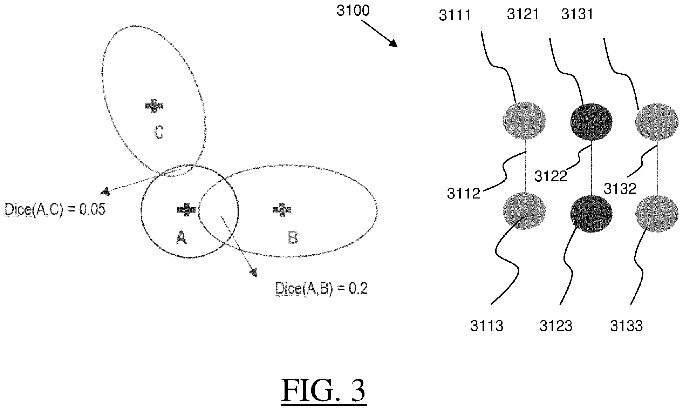

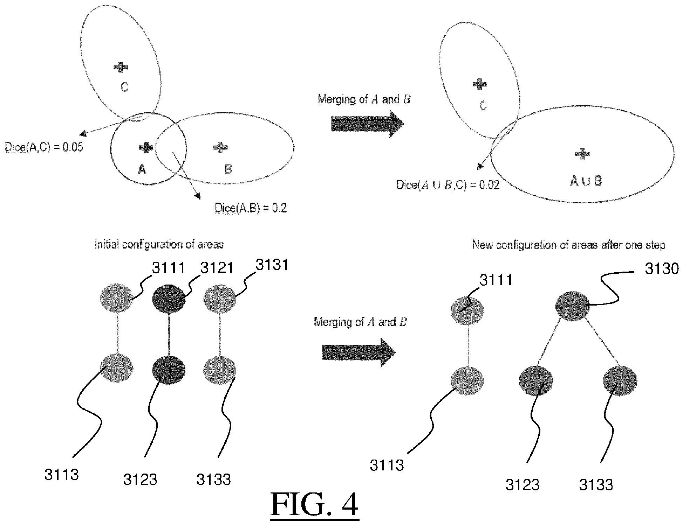

An example is now discussed with reference to FIG. 3 and FIG. 4.

FIG. 3 shows the initialization of the method. The method comprises determining the graph 3100. The nodes of the graph 3111, 3121, 3131 represent first regions, the nodes of the graph 3113, 3123, 3133 represent second regions, the edges 3112, 3122, 3132 represent the respective tractogram cluster connecting respective pair of nodes {3111,3113} {3121,3123}, {3131,3133}. At the initialization, the graph comprises the pairs of nodes {3111,3113} {3121,3123}, {3131,3133} respectively linked with edges 3112, 3122, 3132.

A second stage included in the iterative merging process is now discussed.

In a first iteration, the method may comprise selecting a pair regions, e.g., randomly (e.g., among all pairs of the identified first regions and all pairs of the identified second regions). The stage may comprise determining a value of the dice score between the pair of regions.

FIG. 3 shows a region A corresponding to graph node 3121, a region B corresponding to graph node 3131 and a region C corresponding to graph node 3111. The dice score is defined as twice the number of extremities (represented as voxels) comprised in region A and comprised in region B over the sum of the number of extremities in A and the number of extremities in B. If the dice score is close to 1, it means that areas A and B have a large number of voxels in common. If the dice score is close to 0, it means that areas A and B have few voxels in common. The dice score between regions A and B is Dice(A,B)=0.2. The dice score between regions A and C is Dice (A,C)=0.05.

If the dice score is below the first predetermined threshold, the second stage may comprise proceeding to select another pair of regions. Else, if the dice score is above the first predetermined threshold, the second stage comprises merging the pair of regions, thereby creating a new region. The second stage comprises merging the pair of nodes corresponding to the pair of regions, thereby creating a node representing the new region.

FIG. 4 shows the result of the merging process in the first iteration. The first predetermined threshold may be lower than 0.2 (e.g., 0.1). As the dice score between region A and region B is above the predetermined threshold, the second stage merges the pair of regions into a new region AUB corresponding to graph node 3130 representing the union of region A and region B. Accordingly, the graph representing the regions (the nodes and edges being represented with the same reference numerals) is updated by replacing the nodes of the regions A and B by a new node 3130.

In a second iteration, the second stage may comprise selecting the region resulting from the preceding iteration with another region (e.g., chosen randomly or within a predetermined distance of the resulting region). The second stage may comprise measuring the dice score between the pair of regions. If the dice score is above the first predetermined threshold, the second stage comprises merging the pair of regions. The second stage comprises merging the pair of nodes corresponding to the pair of regions.

The iterative merging process (including the first stage and the further/second stage) may continue to perform the first stage until no pair of regions having a value of the dice score above the first predetermined threshold exists.

This improves the accuracy of the parcellation. Indeed, the graph follows the anatomical hypothesis that the graph representing brain network connectivity should be connected. Thanks to performing the merging of the pair of nodes in addition to merging the pair of regions, the method ensures that the output follows this anatomical hypothesis. In turn, as the regions are located at the interface of grey and white matter, the parcellation provides anatomically relevant information about the grey matter that follows the connectivity given by fibrous white matter in the human brain.

The further/second stage may comprise identifying a principal connected component of the graph. The principal connected component of the graph may be the largest sub-graph of the graph having vertices that are linked through a path of edges. The further stage may also comprise identifying one or more secondary connected components of the graph. The secondary connected components may be the remaining sub-graphs of the graph having vertices that are linked through a path of edges and which are not connected to the principal connected component.

The further stage may comprise performing, iteratively, an explorative process. The explorative process is performed on pairs of a respective node of the principal connected component and a node of a respective secondary connected component. In other words, at each iteration, the explorative process is performed on a node of the principal connected component and a node of the respective secondary connected component. The explorative process may select each pair randomly or according to a sequence or any other type of selection.

The explorative process may comprise, at each iteration for a respective pair, re-determining a value of the metric quantifying an overlap between regions corresponding to the respective pair. In other words, the method calculates the value of the metric between the node of the principal connected component and the node of the respective secondary component.

The explorative process may also comprise merging the node of the respective secondary component with the respective node of a principal connected component having the highest value of the metric quantifying an overlap between the corresponding regions.

For example, the explorative process may select a node of the principal connected component, the node being fixed. The explorative process may select, at each iteration, a node of a respective secondary connected component and determine a respective value of the metric between regions corresponding to a respective pair of nodes (the node of the principal component being fixed while the node of the respective secondary connected component being changed for the iteration). The method may keep track of the value of the metric quantifying the overlap determined at each iteration. After exploring (e.g., all of) the nodes of the respective secondary component, the method merges the node of the respective secondary component with the respective node of a principal connected component having the highest value of the metric. In other words, the method connects the principal connected component with the secondary component by replacing the pair of nodes with the node corresponding to the merging. The graph may be updated to replace the pair of nodes with the node corresponding to the merging, and updating the edges of the graph accordingly. Thereby, the principal connected component is connected to the secondary component.

The explorative process may be performed in parallel: the method may comprise setting threads on pairs of a respective node of the principal connected component and a node of a respective secondary connected component. Thus, the explorative process may for example be performed efficiently on a GPU.

The method may perform the explorative process until there exists no secondary component having a node representing a region forming an overlap for which the value of the metric (quantifying overlap between two regions) is above zero with a region represented by a node of the principal connected component..

This improves theaccuracy of the parcellation. Indeed, this results in that nodes of respective secondary components represent regions overlapping with regions represented by nodes of the principal connected component are merged, and thus the method enforces the anatomical hypothesis that the graph representing the brain network should be connected. At this point, there may still be secondary components which are isolated from the principal connected graph, as there may be secondary components having regions not overlapping with a region represented by a node of the principal connected component (the value of the metric being zero, up to numerical rounding errors).

Determining the parcellation may comprise, after the iterative merging process, performing an iterative post-processing over remaining regions of grey matter.

The iterative post-processing may comprise, at each iteration, determining pairs of overlapping regions, (i.e., that remain from performing the explorative process). The iterative post-processing may also comprise determining a portion representing the overlap between the regions. The portion may for example be represented as one or more voxels.

The iterative post-processing may also comprise computing respective Mahalanobis distances between a respective region of the pair and the determined portion. The Mahalanobis distance may be a measure of the distance between the portion and a distribution defined by the region. The iterative post-processing may also comprise assigning the portion to a respective region of the pair having the smallest Mahalanobis distance with respect to the portion.

This reaches a state where there are no overlapping regions, as the parcellation contains one area per portion. Thereby, the post-processing improves the anatomical hypothesis that the graph representing the brain network should be connected, as secondary components isolated from the principal connected graph are connected.

The respective Mahalanobis distance may be of the type:

$$d_{Maha} = \left\| \frac{x - \mu_{cluster}}{\sigma_{cluster}} \right\|_2,$$

wherein $\mu_{cluster}$ is the mean of voxels of a corresponding region of the pair. $\sigma_{cluster}$ is the standard deviation of voxels of the corresponding region of the pair. and $x$ is a position of a voxel belonging to the predetermined portion.

The respective Mahalanobis distance ensures that voxels belonging to more to one region are assigned to only one region, and thus the output parcellation does not present overlapping voxels, that is, the obtained graph is connected and each voxel at the white-grey matter interface in the brain belongs to one region. Thus, the method fulfils the anatomical hypothesis and the constraint of having one area only in each voxel, thereby being anatomically accurate.

The method may further comprise, after performing the iterative post-processing, determining small regions. Each small region is, by definition, a (first or second) region having an average number of voxels (representing respective first or second extremities) below a second predetermined threshold. The method may for example count the number of (first or second) extremities (e.g., represented by voxels) comprised by a respective (first or second) region, compute an average number of extremities of per region. The second predetermined threshold may be a user-defined threshold times (i.e., multiplied by) the average number. The method determines that the region is a small region if the average number of voxels of said region is below the second predetermined threshold.

The method may also further comprise, for each small region, determining neighboring large regions. A large region is, by definition, a (first or second) region having an average number of voxels above the second predetermined threshold. By neighboring it is meant a spatial neighborhood with respect to the small region.

The method may also further comprise, counting the most present anatomical category among anatomical categories associated to each respective determined neighboring large region. For example, the neighboring large region may be associated to one or more labels, each label having information of a respective anatomical category. The method may count the most present label of a respective anatomical category.

The method may also further comprise, for each small region, assigning voxels (representing first or second extremities) of the small (first or second) region to the respective neighboring large region having the largest number of an anatomical category. In other words, the assignment is voxel-wise to the neighboring large region having the largest number of an anatomical category. The method may proceed voxel per voxel so that the voxels comprised in the small region are assigned to the neighboring large regions. The method may stop the assigning when the small region is empty.

This results in an improvement in the parcellation. Indeed, the method merges small regions with large regions, so that there is no redundant or useless information that may clutter the information already presented (as represented by the labels) in the large regions.

The method has thereby yielded a parcellation of grey matter at the interface with white matter at this stage. This may already be used as described earlier (e.g., displayed and/or for diagnostic/treatment). The method may further comprise determining a parcellation of the whole grey matter based on this (intermediary) parcellation of grey matter at the interface with white matter. This may be performed in any manner. For example, the method may comprise assigning each voxel representing grey matter to its closest region found in the intermediary parcellation. The method may perform the assigning via a distance information between each voxel of the grey matter and its closest labelled voxel found in the intermediary parcellation.

The predetermined clustering algorithm may be any algorithm configured to cluster a plurality of tractogram streamlines based on spatial proximity and/or on shape similarity.

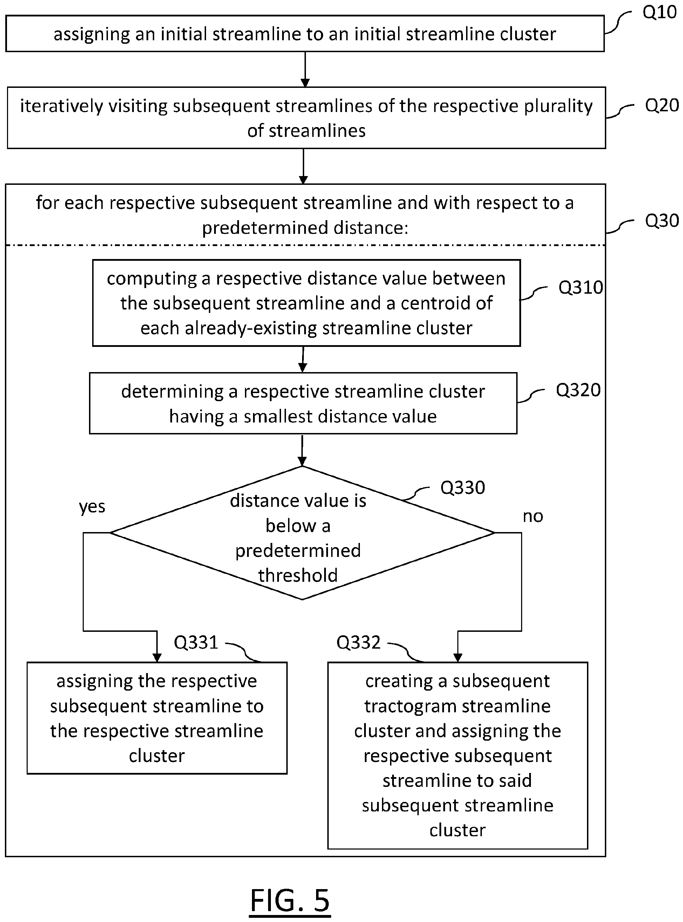

The predetermined clustering algorithm is now further discussed, with reference to FIG. 5.

The predetermined clustering algorithm may take as input a plurality of tractogram streamline. In other words, the predetermined clustering algorithm may be applied to tractogram streamlines of the tractogram to obtain a respective plurality of tractogram streamline clusters. The predetermined clustering algorithm may take as input full-fiber, non-looping and/or sectioned-fiber tractogram streamlines (e.g., only full-fiber tractogram streamlines) and output the plurality of tractogram streamline clusters. In other words, steps of the predetermined clustering algorithm below may be performed on input full-fiber, non-looping and/or sectioned-fiber tractogram streamlines to compute the output tractogram streamline clusters. Accordingly, the output tractogram streamline clusters may comprise full-fiber, non-looping and/or sectioned-fiber tractogram streamlines. For example, at least part (e.g., one or more or all) of the tractogram streamline clusters may comprise only full-fiber tractogram streamlines.

The method may comprise obtaining a threshold value as a third predetermined threshold.

The predetermined clustering algorithm may comprise assigning Q20 an initial tractogram streamline to an initial tractogram streamline cluster. The initial tractogram streamline may be chosen in any manner, for example, it may be randomly chosen from all of the tractogram streamlines. In examples, the initial tractogram streamline may be chosen according to an indexation (e.g., the indexation being ordered) of the tractogram streamlines determined when obtaining the tractogram.

The predetermined clustering algorithm may also comprise, iteratively visiting Q30 subsequent tractogram streamlines of the respective plurality of tractogram streamlines. In other words, the predetermined clustering algorithm may traverse all other tractogram streamlines not having been chosen as the initial tractogram streamline. The visiting may be performed in any manner, for example, if the tractogram streamlines are indexed, the method may visit iteratively the indices (e.g., one-by-one) of tractogram streamlines subsequent (e.g., above or below) from the initial tractogram streamline.

For each respective subsequent tractogram streamline and with respect to a predetermined distance Q40, the predetermined clustering algorithm may also comprise computing Q410 a respective distance value between the subsequent tractogram streamline and a centroid of each already-existing tractogram streamline cluster.

By "respective subsequent tractogram streamline" it is meant a tractogram streamline visited by the predetermined clustering algorithm. By "already-existing" tractogram streamline cluster it is meant any tractogram streamline cluster created beforehand by the predetermined clustering algorithm, for example the initial tractogram streamline cluster when there is no other tractogram streamline cluster.

The centroid may be a mean tractogram streamline of a respective tractogram streamline cluster with respect to other tractogram streamlines already included (assigned, e.g., via a label) to the cluster. That is, the centroid may be a tractogram streamline (being for example a sequence of 3D points) assigned (i.e., included) in the tractogram streamline cluster which satisfies an average position with respect to other tractogram streamlines included in the cluster. The average position may be with respect to an average distance (for instance with respect to the same predetermined distance) with respect to other tractogram streamlines already included in the cluster, e.g., the centroid may be the tractogram streamline extending in the geometrical average (within some rounding errors) with respect to other tractogram streamlines already included in the cluster (for example the initial tractogram streamline in the case where the algorithm visits a subsequent tractogram streamline after the assigning Q20). The average may be a numerical average, for example, when the tractogram streamlines are indexed in the cluster. The centroid of each tractogram streamline cluster may be dynamically re-positioned when a tractogram streamline is assigned to the tractogram streamline cluster, that is, at each time when a new tractogram streamline is assigned to the tractogram streamline cluster.

The computing Q410 may be the result of computing the respective predetermined distance between the subsequent tractogram streamline and the centroid, for each already existing cluster.

Examples of the predetermined distance are now discussed.

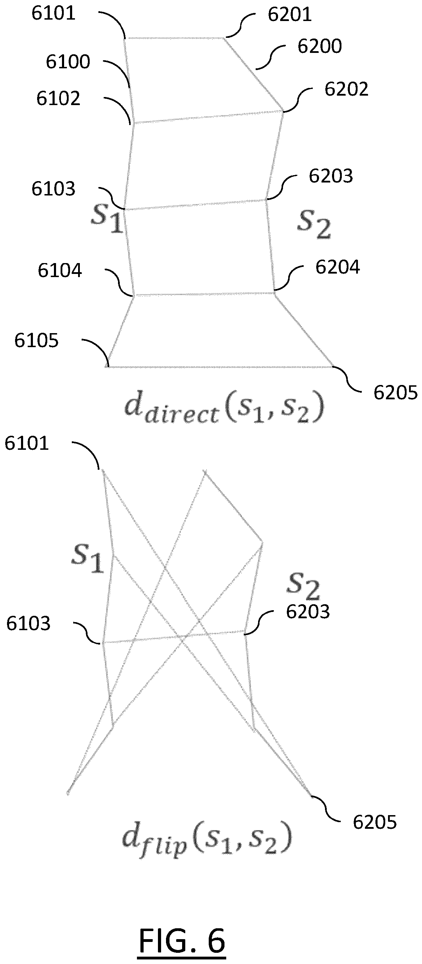

The predetermined distance may be a minimum direct-flip distance. Reference is made to FIG. 6, illustrating, on two tractogram streamlines 4100, 4200, the case where the predetermined distance may be the minimum direct-flip distance of the type:

$$MDF = \min\left(d_{direct}(f,s), d_{flip}(f,s)\right),$$

Where the predetermined distance is the minimum of the terms $d_{flip}(f,s) = d(f,s^F) = d(f^F,s)$ and $d_{direct} = \max(\text{dist}_{eucl}(s_{1_{points}}, s_{2_{points}}))$, where $f,s$ denote two points of input tractogram streamlines $s_1$ 6100 and $s_2$ 6200.

The term $d_{direct}$ denotes the maximum of the Euclidean distance (also called direct distance) between points $(s_{1_{points}}, s_{2_{points}})$ of the two tractogram streamlines $s_1$ and $s_2$. The points $(s_{1_{points}}, s_{2_{points}})$ may be obtained in any manner, for example by performing a same discretization of the two tractogram streamlines (uniform or not-uniform, however the same number of points for the two tractogram streamlines), or by sampling points from the two tractogram streamlines $s_1$ and $s_2$. The points may be ordered and the method may compare the distance between points having the same order. For example, the two tractogram streamlines $s_1$ and $s_2$ may have an ordered sequence of points 6101 - 6105 (for the tractogram streamline $s_1$) and 6201-6205 (for the tractogram streamline $s_2$). The direct distance $d_{direct}$ may thus compute the maximum of the Euclidean distance between two points, e.g., (6101,6201), (6102,6202) and so forth.

The term $d_{flip}(f,s)$ denotes a flip-distance term. Denoting as $f,s$ two points of each tractogram streamline, and $s^F$, $f^F$ two points corresponding to a flip in each respective tractogram streamline (or permutation in each respective tractogram streamline), the flip-distance term has the property $d_{flip}(f,s^F) = d_{flip}(f^F,s)$; in other words, the flip-distance between $f$ and $s^F$ is the same as the distance between $f^F$ and s. For example, the term $d_{flip}(f,s)$ may compute the distances between two extremum points (4101,4205) for example, the points corresponding to the first and last point respectively of each tractogram streamline $s_1$ and $s_2$, and may also compute the distances between two center points (4103,4203), that is the points corresponding to middle points of each tractogram streamline $s_1$ and $s_2$ and so forth.

The predetermined clustering algorithm may also comprise, determining Q420 a respective tractogram streamline cluster having a smallest distance value (with respect to the respective subsequent tractogram streamline) among the respective distance values computed at Q410. In the case where there are more than one tractogram streamline clusters having the smallest distance value (e.g., equidistant to the subsequent tractogram streamline up-to some tolerance), the method may chose randomly the respective tractogram streamline cluster among said tractogram streamline clusters.

If the respective (smallest) distance value is below the third predetermined threshold Q430, the predetermined clustering algorithm may assign Q431 the respective subsequent tractogram streamline to the respective tractogram streamline cluster. For example, the assigning Q431 may associate the respective subsequent tractogram streamline to a data piece (e.g., such as a label) indicative of its assigning with the respective tractogram streamline cluster being determined as having the smallest distance value at Q420.

Else, (that is, the respective smallest distance is above the third predetermined threshold) the predetermined clustering algorithm may create Q432 a subsequent tractogram streamline cluster and assign the respective subsequent tractogram streamline to said subsequent tractogram streamline cluster. For example, the creating Q432 may associate the respective subsequent tractogram streamline to a data piece (e.g., such as a label) indicative of its assignation to a new cluster, i.e., part of a new set of tractogram streamlines, and thus being discriminated from other tractogram streamline clusters.

The method thus improves on the segmentation. Thanks to using the predetermined clustering algorithm, the method reduces the dimensionality of the tractogram by gathering tractogram streamlines that have a similar shape (as represented by the fulfillment of the predetermined distance) and a close location.

Moreover, when the predetermined distance is the minimum direct-flip distance, this results in that the method improves the quality of the clusters, as the predetermined clustering algorithm dissociates tractogram streamlines which are close in the middle and which separate significantly around its extrema.

The predetermined clustering algorithm may further comprise, after assigning all tractogram streamlines of the plurality of tractogram streamlines, re-computing the centroid of each tractogram streamline cluster using the predetermined centroid computation algorithm. In other words, the predetermined centroid computation algorithm may compute again a mean tractogram streamline of the tractogram streamline cluster with respect to the currently assigned tractogram streamlines. Indeed, after all the successive assignments, the centroid may have changed. Re-computing the centroid at such a time, to perform the following steps, allows a compromise between computational costs and final accuracy, as explained in the following.

The predetermined clustering algorithm may also comprise, for each tractogram streamline assigned to a respective tractogram streamline cluster, and with respect to the predetermined distance, computing a respective distance value between the tractogram streamline and the centroid of the respective tractogram streamline cluster. For example, the predetermined distance may be the minimum direct-flip distance. Additionally, the predetermined clustering algorithm may also un-assign the tractogram streamline from the respective tractogram streamline cluster if the respective distance value is above the predetermined threshold. In other words, the un-assigning removes the association of the tractogram streamline associate with the respective tractogram streamline cluster, e.g., by removing the data piece (e.g., such as a label) indicative of its assignation, or marking it with another data piece indicative of not being assigned to the respective tractogram streamline cluster.

The predetermined clustering algorithm may also comprise re-computing again the centroid of each tractogram streamline cluster using the predetermined centroid computation algorithm. In other words, the predetermined centroid computation algorithm may compute the mean tractogram streamline of the tractogram streamline cluster with respect to tractogram streamlines that remained assigned to the respective tractogram streamline cluster after the un-assigning.

The predetermined clustering algorithm may also comprise, for each tractogram streamline un-assigned to another respective tractogram streamline cluster, and with respect to the predetermined distance, computing a respective distance value (e.g., the minimum direct-flip distance) between the tractogram streamline and the centroid of each tractogram streamline cluster.

The predetermined clustering algorithm may also determine a tractogram streamline cluster having a smallest distance value. If the respective distance value is below the predetermined threshold (e.g., of 5mm or less), the predetermined clustering algorithm may re-assign the unassigned tractogram streamline to the determined tractogram streamline cluster. In the case where there are more than one tractogram streamline clusters having the same smallest distance value, the method may re-assign the unassigned tractogram streamline to one of said tractogram streamline clusters chosen randomly. Else, the predetermined clustering algorithm may create a subsequent tractogram streamline cluster and re-assign the subsequent tractogram streamline to a subsequent tractogram streamline cluster.

This further improves the quality of the segmentation of the tractogram. Indeed, the predetermined clustering algorithm allows to reallocate tractogram streamlines which may have been misplaced, thereby resulting in a finer control on how the tractogram streamlines are clustered. Indeed, before the re-computing of the centroid of each tractogram streamline cluster using the predetermined centroid. The predetermined clustering algorithm including the re-computation of centroids results in a finer discretization of the tractogram streamlines where each tractogram streamline is allocated to a different cluster if it does not correspond to its original cluster.

The predetermined clustering algorithm may obtain another threshold value. The other threshold value is higher than the threshold value that is set as the third predetermined threshold obtained at Q10. Additionally, prior to the steps of using the predetermined clustering algorithm and to the steps of selecting the one or more first sets and the one or more second sets, the method may comprise retrieving P10 all the tractogram streamlines from the tractogram. In other words, the method collects the set of all tractogram streamlines for any (e.g., all of) steps P20-P40 below.

The method may also comprise applying P20 the predetermined clustering algorithm to said (retrieved) all the tractogram streamlines to obtain an initial plurality of tractogram streamline clusters. The initial plurality of tractogram streamline clusters may be a set of initial tractogram clusters, for example, not being previously associated with any other cluster.

The method may also comprise selecting P40 one or more initial sets of tractogram streamlines from the plurality of initial tractogram streamline clusters. The one or more initial sets of tractogram streamlines selected in P40 may be the ones on which the attributing S20 is performed. A respective initial tractogram streamline cluster may be selected as an initial set when the respective initial tractogram streamline cluster fulfills a coarser proximity criterion. By "coarser proximity criterion" it is meant that the initial tractogram clusters have a larger distance compared to the result of applying the steps of using the predetermined clustering algorithm at steps Q10-Q40. The higher predetermined threshold may be the input threshold value. For example, the predetermined threshold used at S20 may be of 5mm while the coarser proximity criterion may have a threshold of 15mm.

The using of the predetermined clustering algorithm (with the threshold value being lower than the other threshold value as the third predetermined threshold at Q10) to obtain the plurality of tractogram streamline clusters may be applied to the streamlines of at least part (e.g., all of) of the one or more initial sets of tractogram streamlines. The using of the predetermined clustering algorithm may determine positions on the tractogram from which the at least part of the or more initial sets of tractogram streamlines are obtained, for example, following an initial distribution of said positions or a totally random distribution of said positions.

This improves the efficiency of the segmentation. As the initial tractogram streamline clusters are selected as initial sets when the respective initial tractogram streamline cluster fulfills the coarser proximity criterion, the selected one or more initial sets are a rough (or coarse) first segmentation that removes tractogram streamlines, for example, outlier tractogram streamlines or which are not located on a particular zone of interest.

Examples of the method are now discussed with reference to FIG.s 7 to 13.

In an example implementation, the method is used to provide an anatomical parcellation of a subject's brain based on the white matter fibrous structure of the brain. The output parcellation may be called a "connectivity-based" parcellation where each voxel of the grey matter must be associated to a single cortical or subcortical area.

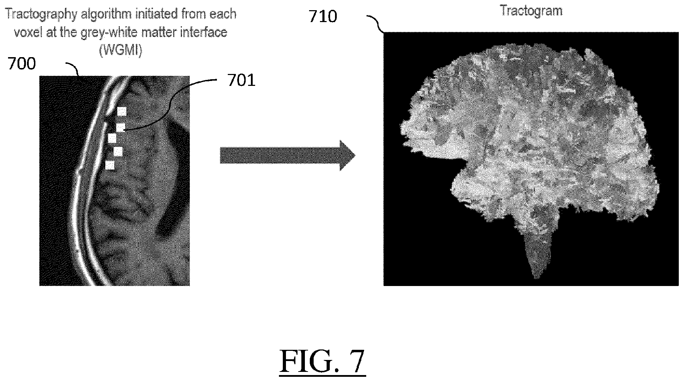

FIG. 7 illustrates the input of the method.

*Step 510*

The method may model the white matter fibrous structure of the brain using a tractography algorithm on an input diffusion MRI 700. The method initiates a tractography algorithm using seed voxels 701 located at the interface of the white and grey matter in the brain.

The method obtains a tractogram 710 from the input diffusion MRI 700. For example, the method may comprise determining diffusion voxel model from the diffusion MRI (e.g., obtained from a Fiber Orientation Density Function). The method may obtain the tractogram from the diffusion voxel model as discussed above.

*Step S20*

Once the method obtains the tractogram (ensemble of fibers), the method may apply a predetermined clustering algorithm on the tractogram to obtain tractogram streamline clusters. The clustering algorithm is configured to assemble fibers that have a similar shape and location in space into a single cluster. In other words the predetermined clustering algorithm reduces the dimensionality of the tractogram. In fact, a tractogram contains up to millions of streamlines and the clustering algorithm allows reducing this number to around 3000 clusters, each tractogram streamline cluster being described by a centroid, which is the mean streamline of all streamlines within a tractogram streamline cluster.

*Step S30*

Once the streamlines have been gathered into clusters, the method identifies a respective first region of grey matter including for each tractogram streamline of the cluster its first extremity, and a respective second region of grey matter including for each tractogram streamline of the cluster its second extremity the method identifies voxels located at the endpoints of each cluster then separates the endpoints of the first and second regions using a k-means algorithm with k=2.

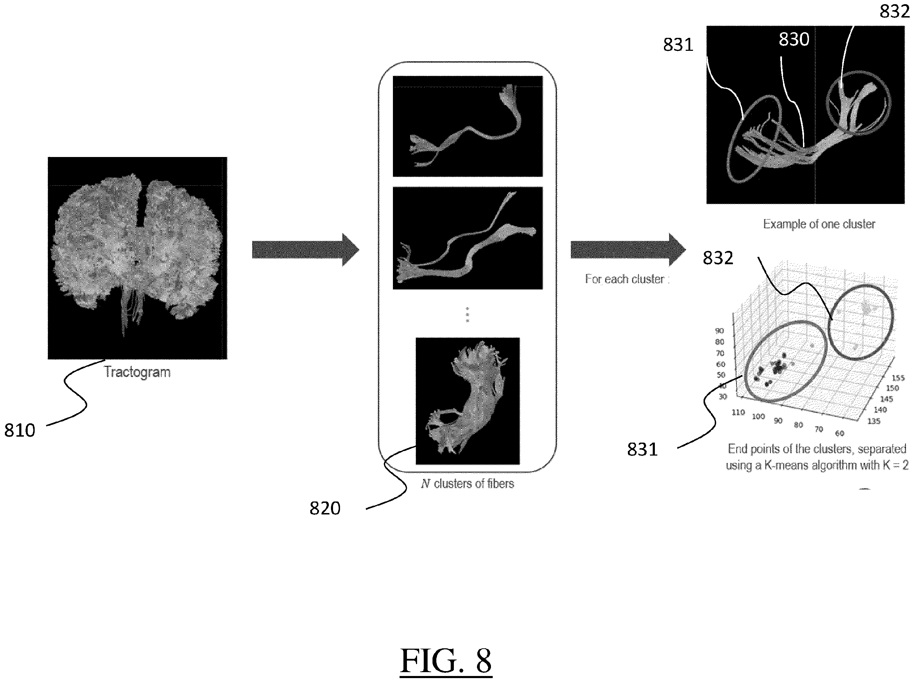

FIG. 8 illustrates the identification of respective first and second regions.

The method comprises obtaining the tractogram of the brain 810 (S10). The method uses the predetermined clustering algorithm (S20) to obtain the plurality 820 of tractogram streamline clusters (N clusters of tractogram streamlines/fibers). For each tractogram streamline cluster 830, the method identifies (S30) a respective first region 831 of grey matter including for each tractogram streamline of the cluster its first extremity, and a respective second region 832 (separate of the first region) of grey matter including for each tractogram streamline of the cluster its second extremity. The extremities (or endpoints) 831,832 may be represented as 3D points (or voxels). The method identifies the regions by using a k-means algorithm with k=2.

The identification by the method, for each cluster, of the ensemble of voxels describing the two end areas of the clusters allows having a first connectivity-based parcellation. This initial connectivity-based parcellation will be processed by the method as some areas share voxels, those are called overlapping areas.

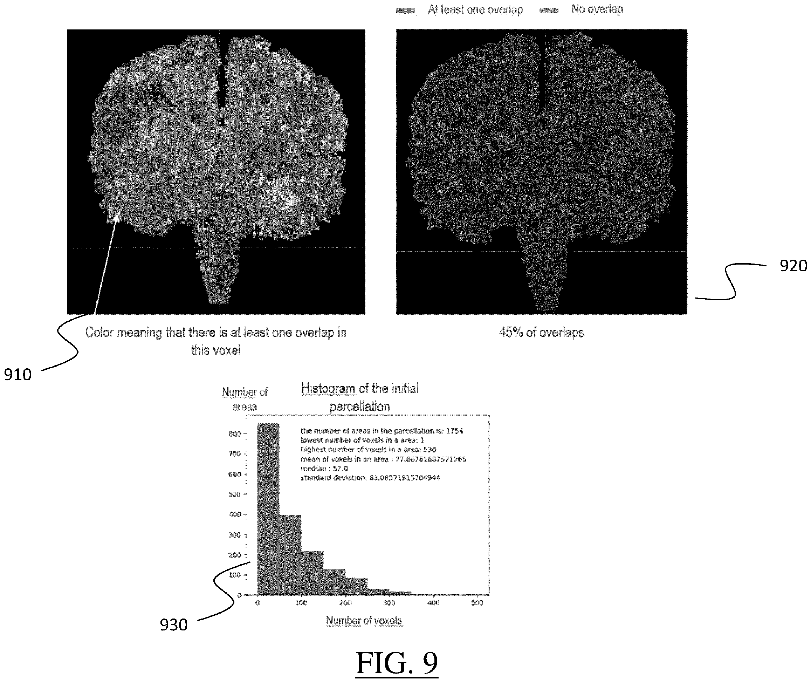

FIG. 9 shows the output 910 of the identification of the first regions and second regions. The output shows that a 45% of voxels are overlapped by regions 920. The histogram 930 shows the histogram of this initial parcellation. The number of areas in the parcellation of this example is: 1754, the lowest number of voxels in a region is 1, the highest number of voxels in a region is 530. The mean of voxels in an area is: 77.66761687571265, the median is 52.0 and the standard deviation is 83.08571915704944.

The method introduces the pre-determined clustering algorithm as an initialization of the connectivity-based parcellation method. In fact, the pre-determined clustering algorithm allows reducing the computation time and initiating a connectivity-based parcellation on the entire brain without any external anatomical knowledge. Thus, it allows having a global method at a reduced computation cost.

*Step 540*

The processing by the method of the obtained initial connectivity based parcellation relies on an iterative process using dice scores and graph information.

The initialization steps are the following:

- for each pair of overlapping areas, computing the dice score between the two considered areas.

- build a graph of the area where the nodes are the cortical or sub-cortical areas and the edges are the clusters between areas. At the initialization, the graph is made of multiple pairs of nodes, representing the two end areas of a cluster.

The method comprises the following iterative steps:

- sorting the dice scores, and the corresponding pair of overlapping areas, from the biggest (closest to 1) to the smallest (closest to 0)
- getting the maximum dice score and its corresponding pair of overlapping areas and check if the dice score is above a user-defined threshold, if it is above the threshold:

  ▪ merge the pair of overlapping areas, corresponding to the highest dice score, into a new region,
  ▪ update the graph to create a new node corresponding to the pair of overlapping areas that has been merged, update the edges of the graph accordingly,
  ▪ compute the dice score between the newly created area and all areas that it overlaps,
  ▪ go back to the first step,

If it is not above the threshold, stop the iterative steps

After the iterative steps, the number of areas have been considerably reduced compared to the initial number of areas and the graph is composed of the corresponding reduced number of nodes. However, some voxels are still shared between pairs of overlapping areas. As the final connectivity-based parcellation should only contain one area per voxel, the method treats the final pair of overlapping areas and a criterion for that.

The method uses an anatomical hypothesis for the post-processing of the remaining pairs of overlapping areas and the corresponding graph. The anatomical hypothesis states that the graph, representing the brain network, should be connected.

Thus, the next step of the method is to test the connections of the graph by performing:

- If the graph is connected, go to the final post-processing step
- If the graph is not connected, some areas must be merged by performing:

    ○ getting the areas that constitute the main connected component from the graph and isolate the areas that are not connected to the main component of the graph
    ○ for each area that is not part of the main component of the graph, computing the dice score between the considered area and its overlapping areas belonging to the main component of the graph,
    ○ finding the pair of overlapping areas that corresponds to the highest dice score
    ○ merging the selected pair of overlapping areas,
    ○ updating the graph to create a new node corresponding to the pair of overlapping areas that has been merged, update the edges of the graph accordingly,
    ○ checking if the graph is connected and go back to the iterative steps,

After the steps above, there exists no secondary component having a node representing a region forming an overlap for which the value of the metric is above zero with a region represented by a node of the principal connected component.

It is possible that some areas are still overlapping and share voxels. As the final connectivity-based parcellation must have only one area per voxel, the method treats voxels belonging to more than one area in a post-processing step.

For each voxel that belong to more than one area, the method computes Mahalanobis distances between the considered voxel and each area it belongs to, the voxel is then assigned to the area which has the smallest Mahalanobis distance with the considered voxel. The Mahalanobis distance is the following:

$$d_{Maha} = \left\| \frac{x - \mu_{cluster}}{\sigma_{cluster}} \right\|_2$$

where $\mu_{cluster}$ is the mean of the cluster voxels; $\sigma_{cluster}$ is the standard deviation of the cluster voxels and x is the considered voxel position.

After these steps, the method obtains a connected graph and each voxel of the white-grey matter interface within the entire brain belongs to only one area. Thus, the connectivity-based parcellation fulfils the anatomical hypothesis and the constraint of having one area only in each voxel.

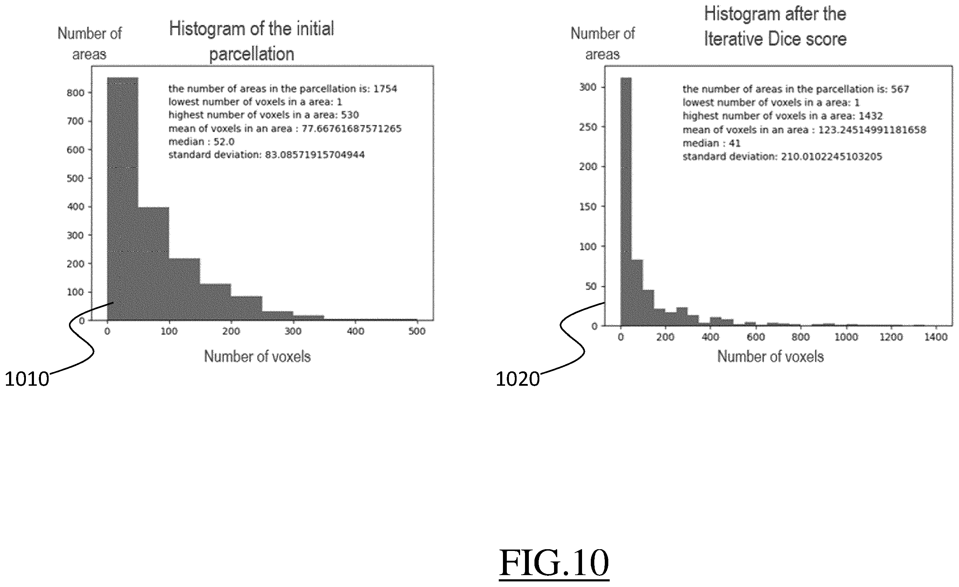

FIG. 10 shows a histogram 1010 of the initial connectivity-based parcellation and a histogram 1020 after performing the second stage of merging pairs of nodes of the graph.

Some areas are much smaller than others and a post-processing step has been implemented as an optional part of the pipeline:
For each area, the post-processing comprises:

- counting the number of voxels,
- computing the average number of voxels per area,
- setting a user-defined threshold to a threshold times (multiplied by) the average number of voxels per area,
- finding the areas whose number of voxels is below the user-defined threshold,
- getting the neighbors of the considered area whose number of voxels is above the user-defined threshold,
- counting the most present label in the neighbors found,
- merging the small area with the most present area,
- updating the graph accordingly.

Finally, the connectivity-based parcellation is obtained only on the grey-white matter interface voxels. There is a need to label the entire grey matter from the obtained parcellation. This label transfer can be performed by the method by using a distance information between each voxel of the grey matter and its closest labelled voxel.

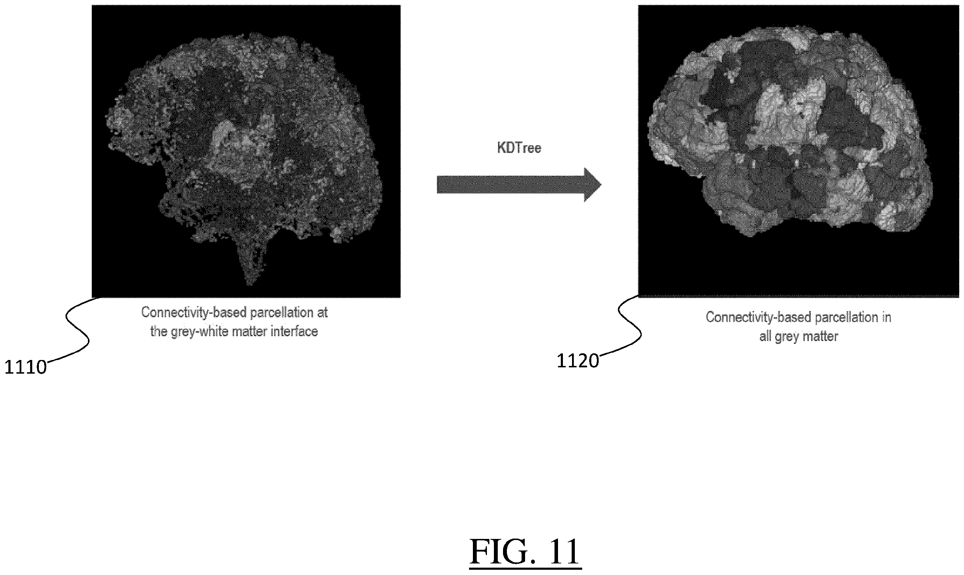

FIG. 11 shows the parcellation 1110 prior to the post-processing and the parcellation after 1120 the post processing.

The output by the method may be validated by comparison with known functional and anatomical areas to the connectivity-based parcellation obtained, i.e., ground truth.

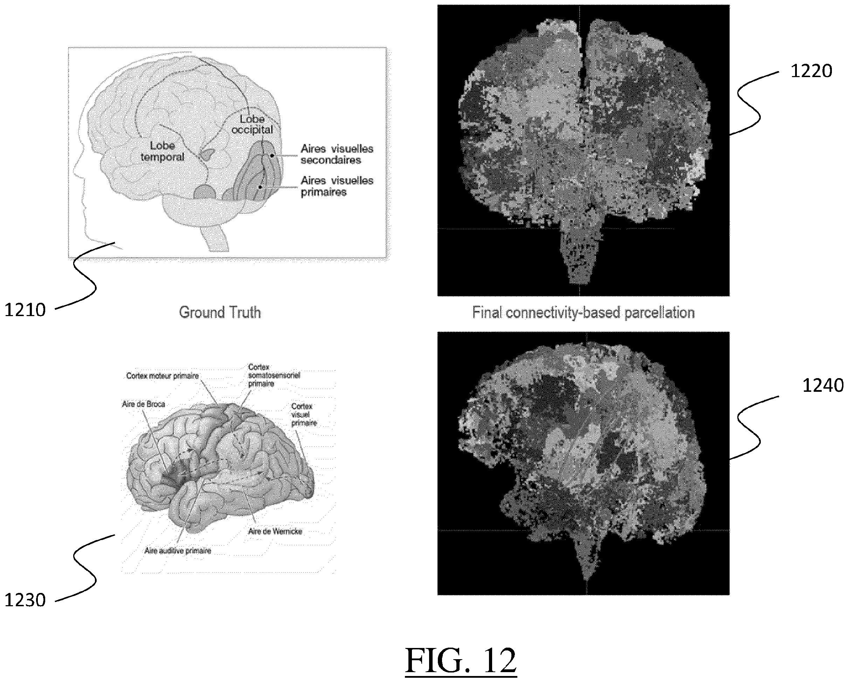

FIG. 12 shows ground truths 1210, 1230 compared with outputs 1220, 1240.

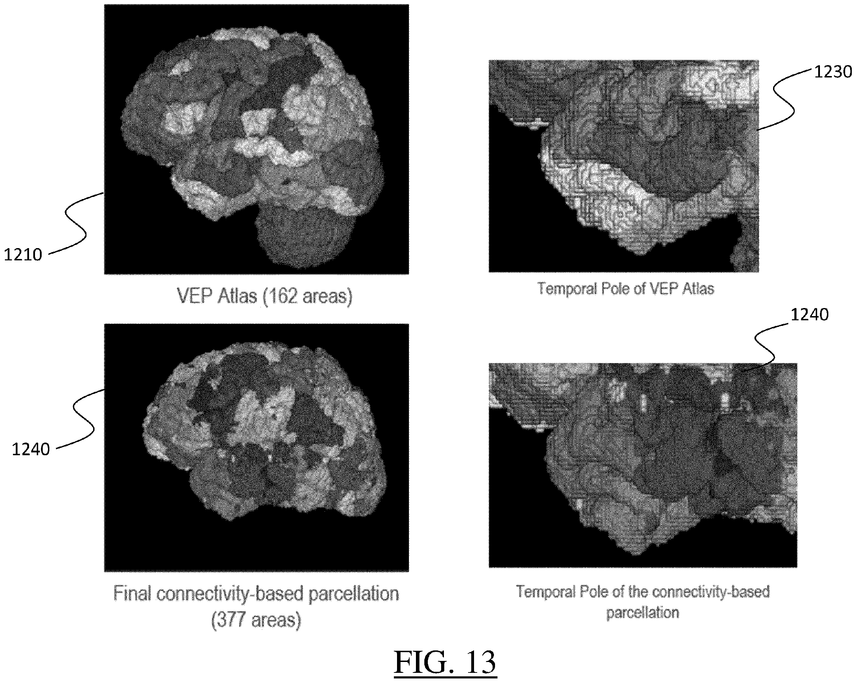

FIG. 13 shows ground truths 1310, 1330 compared with outputs 1320, 1340.

**Claims**

**1.** A computer-implemented method for parceling grey matter of a brain of a human patient, comprising:

- obtaining a tractogram of the brain of the human patient, the tractogram including tractogram streamlines, each streamline having a first extremity located in a first portion of grey matter of the brain of the human patient and a second extremity located in a second portion of grey matter of the brain of the human patient, the first portion and the second portion being separate;
- using a predetermined clustering algorithm to obtain a plurality of tractogram streamline clusters;
- for each tractogram streamline cluster of at least a part of the plurality of tractogram streamline clusters, identifying a respective first region of grey matter including for each tractogram streamline of the cluster its first extremity, and a respective second region of grey matter including for each tractogram streamline of the cluster its second extremity, the respective first region being separate from the respective second region;
- determining a parcellation based on the identified regions, the determining of the parcellation including an iterative merging process, the iterative merging process including, at each iteration, merging pairs of regions based on a metric quantifying an overlap.

2. The method of claim 1, wherein the metric quantifying an overlap is a dice score.

3. The method of claim 1 or 2, wherein the iterative merging process includes a stage comprising, at each iteration:

- for each pair of regions, determining a value of the metric, the value of the metric thereby quantifying an overlap between the pair of regions;
- merging one or more respective pairs of regions each having a value of the metric above a first predetermined threshold, for example a pair of regions having a highest value of the metric among all pairs of regions;

the stage of the iterative process being performed until no pair of regions having a value of the metric above the first predetermined threshold exists.

4. The method of claim 3, wherein the method further comprises:

- prior to the iterative merging process, determining a graph comprising, for each tractogram streamline cluster, a first node representing the respective first region and a second node representing the respective second region, and an edge connecting the first node and the second node, the edge thereby representing a respective tractogram streamline cluster,
- at each iteration of the stage, merging each pair of nodes of the graph representing a respective merged pair of regions; and
- after the stage, evaluating whether the graph is connected, wherein if the graph is not connected, the iterative merging process includes a further stage comprising iteratively merging one or more pairs of regions and each pair of nodes of the graph representing a merged pair of regions.

5. The method of claim 4, wherein the further stage comprises:

- identifying a principal connected component of the graph and one or more secondary connected components of the graph; and
- performing, iteratively, an explorative process on pairs of a respective node of the principal connected component and a node of a respective secondary connected component, the explorative process performing at each iteration for a respective pair:

-- re-determining a value of the metric quantifying an overlap between regions corresponding to the respective pair, and
-- merging the node of the respective secondary component with the respective node of a principal connected component having the highest value of the metric quantifying an overlap between the corresponding regions, the explorative process being performed until there exists no secondary component having a node representing a region forming an overlap for which the value of the metric is above zero with a region represented by a node of the principal connected component.

6. The method of any one of claims 1 to 5, wherein determining the parcellation comprises, after the iterative merging process, performing an iterative post-processing over remaining regions of grey matter, the iterative post-processing comprising, at each iteration:

- determining pairs of overlapping regions, and determining a portion representing the overlap between the regions;
- computing respective Mahalanobis distances between a respective region of the pair and the determined portion; and
- assigning the portion to a respective region of the pair having the smallest Mahalanobis distance.

7. The method of claim 6, wherein the respective Mahalanobis distance is of the type:

$$d_{Maha} = \left\| \frac{x - \mu_{cluster}}{\sigma_{cluster}} \right\|_2 ,$$

wherein $\mu_{cluster}$ is the mean of voxels of a corresponding region of the pair, $\sigma_{cluster}$ is the standard deviation of voxels of the corresponding region of the pair, and x is a position of a voxel belonging to the predetermined portion.

8. The method of claim 6 or 7, further comprising, after performing the iterative post-processing:

   - determining small regions, each small region being a region having an average number of voxels below a second predetermined threshold;
   - for each small region:

      -- determining neighboring large regions, a large region being a region having an average number of voxels above the second predetermined threshold,
      -- counting a most present anatomical category among anatomical categories associated to each respective determined neighboring large region;
      -- assigning voxels of the small region to the respective neighboring large region having the largest number of an anatomical category.

9. The method of any one of claims 1 to 8, wherein the predetermined clustering algorithm comprises for a respective plurality of tractogram streamlines:

   • obtaining (Q10) a threshold value as a third predetermined threshold;
   • assigning (Q20) an initial tractogram streamline to an initial tractogram streamline cluster;
   • iteratively visiting (Q30) subsequent tractogram streamlines of the respective plurality of tractogram streamlines; and
   • for each respective subsequent tractogram streamline and with respect to a predetermined distance (Q40):

      ◦ computing (Q410) a respective distance value between the subsequent tractogram streamline and a centroid of each already-existing tractogram streamline cluster; and
      ◦ determining (Q420) a respective tractogram streamline cluster having a smallest distance value:

      ▪ if the respective distance value is below the third predetermined threshold (Q430), assigning (Q431) the respective subsequent tractogram streamline to the respective tractogram streamline cluster;
      ▪ else, creating (Q432) a subsequent tractogram streamline cluster and assigning the respective subsequent tractogram streamline to said subsequent tractogram streamline cluster.

10. The method of claim 9, wherein the predetermined clustering algorithm further comprises, after assigning all tractogram streamlines of the plurality of tractogram streamlines:

   • re-computing the centroid of each tractogram streamline cluster using the predetermined centroid computation algorithm; and
   • for each tractogram streamline assigned to a respective tractogram streamline cluster, and with respect to the predetermined distance:

      ◦ computing a respective distance value between the tractogram streamline and the centroid of the respective tractogram streamline cluster;
      ◦ un-assigning the tractogram streamline from the respective tractogram streamline cluster if the respective distance value is above the third predetermined threshold;

   • re-computing again the centroid of each tractogram streamline cluster using the predetermined centroid computation algorithm; and
   • for each tractogram streamline un-assigned to another respective tractogram streamline cluster, and with respect to the predetermined distance:

      ◦ computing a respective distance value between the tractogram streamline and the centroid of each tractogram streamline cluster;
      ◦ determining a tractogram streamline cluster having a smallest distance value:

      ▪ if the respective distance value is below the third predetermined threshold, re-assigning the unassigned tractogram streamline to the determined tractogram streamline cluster;
      ▪ else creating a subsequent tractogram streamline cluster and re-assigning the subsequent tractogram streamline to a subsequent tractogram streamline cluster.

11. The method of any claim 9 or 10, wherein the predetermined distance is a minimum direct-flip distance.

12. The method of any one of claims 9 to 11, wherein the predetermined clustering algorithm obtains another threshold value, the other threshold value being higher than the threshold value, and prior to the steps of using the predetermined clustering algorithm, the method comprises:

   • retrieving (P10) all the tractogram streamlines from the tractogram;
   • applying (P20) the predetermined clustering algorithm to said all the tractogram streamlines to obtain an initial plurality of tractogram streamline clusters;
   • selecting (P40) one or more initial sets of tractogram streamlines from the plurality of initial tractogram streamline clusters, a respective initial tractogram streamline cluster being selected as an initial set, the respective initial tractogram streamline cluster thereby fulfilling a coarser proximity criterion;

   the using of the predetermined clustering algorithm to obtain the plurality of tractogram streamline clusters being applied to the streamlines of at least part of the one or more initial sets of tractogram streamlines.

13. A computer program comprising instructions for performing the method of any of claims 1-12.

14. A computer readable storage medium having recorded thereon the computer program of claim 13.

15. A system comprising a processor coupled to a memory, the memory having recorded thereon the computer program of claim 13.

obtaining a tractogram of the brain of the human patient — S10

using a predetermined clustering algorithm to obtain a plurality of tractogram streamline clusters — S20

for each tractogram streamline cluster of at least a part of the plurality of tractogram streamline clusters, identifying a respective first region of grey matter including for each tractogram streamline of the cluster its first extremity, and a respective second region of grey matter including for each tractogram streamline of the cluster its second extremity — S30

determining a parcellation based on the identified regions, the determining of the parcellation including an iterative merging process — S40

## FIG. 1

2000

2010

CPU

RAM — 2070

1020

Mass storage
devices controler

Display — 2080

2030 — Hard
drive

B
U
S

Haptic
device — 2090

Network Adapter

Video
RAM — 2100

2050 —

Network

2060 —

GPU

2110

## FIG. 2

Dice(A,C) = 0.05

Dice(A,B) = 0.2

3100

3111    3121    3131

3112    3122    3132

3113    3123    3133

# FIG. 3

Dice(A,C) = 0.05

Dice(A,B) = 0.2

Merging of $A$ and $B$

Dice($A \cup B$,C) = 0.02

A ∪ B

Initial configuration of areas

3111  3121  3131

3113  3123  3133

Merging of $A$ and $B$

New configuration of areas after one step

3111  3130

3113  3123  3133

FIG. 4

assigning an initial streamline to an initial streamline cluster — Q10

iteratively visiting subsequent streamlines of the respective plurality of streamlines — Q20

for each respective subsequent streamline and with respect to a predetermined distance: — Q30

computing a respective distance value between the subsequent streamline and a centroid of each already-existing streamline cluster — Q310

determining a respective streamline cluster having a smallest distance value — Q320

distance value is below a predetermined threshold — Q330

yes / no

assigning the respective subsequent streamline to the respective streamline cluster — Q331

creating a subsequent tractogram streamline cluster and assigning the respective subsequent streamline to said subsequent streamline cluster — Q332

## FIG. 5

$$d_{direct}(s_1, s_2)$$

$$d_{flip}(s_1, s_2)$$

FIG. 6

Tractography algorithm initiated from each voxel at the grey-white matter interface (WGMI)

700

701

710

Tractogram

FIG. 7

# FIG. 8

810 Tractogram

820 N clusters of fibers

For each cluster:

831 End points of the clusters, separated using a K-means algorithm with K = 2

832

831

830

832

Example of one cluster

At least one overlap ▬▬▬ No overlap

Color meaning that there is at least one overlap in this voxel

910

45% of overlaps

920

Number of areas

Histogram of the initial parcellation

the number of areas in the parcellation is: 1754
lowest number of voxels in a area: 1
highest number of voxels in a area: 530
mean of voxels in an area : 77.66761687571265
median : 52.0
standard deviation: 83.08571915704944

Number of voxels

930

<u>FIG. 9</u>

Number of areas

## Histogram of the initial parcellation

800

700

600

500

400

300

200

100

0

0    100    200    300    400    500

Number of voxels

the number of areas in the parcellation is: 1754
lowest number of voxels in a area: 1
highest number of voxels in a area: 530
mean of voxels in an area : 77.66761687571265
median : 52.0
standard deviation: 83.08571915704944

1010

Number of areas

## Histogram after the Iterative Dice score

300

250

200

150

100

50

0

0    200    400    600    800    1000    1200    1400

Number of voxels

the number of areas in the parcellation is: 567
lowest number of voxels in a area: 1
highest number of voxels in a area: 1432
mean of voxels in an area : 123.24514991181658
median : 41
standard deviation: 210.0102245103205

1020

## FIG.10

Connectivity-based parcellation at the grey-white matter interface

1110

KDTree

Connectivity-based parcellation in all grey matter

1120

FIG. 11

FIG. 12

1240

Final connectivity-based parcellation
(377 areas)

1210

VEP Atlas (162 areas)

Temporal Pole of the connectivity-based
parcellation

1240

Temporal Pole of VEP Atlas

1230

FIG. 13

Europäisches
Patentamt

European
Patent Office

Office européen
des brevets

# EUROPEAN SEARCH REPORT

**Application Number**

EP 24 30 6171

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SILVA FELIPE ET AL: "Cortical Surface Parcellation Based on Graph Representation of Short Fiber Bundle Connections", 2019 IEEE 16TH INTERNATIONAL SYMPOSIUM ON BIOMEDICAL IMAGING (ISBI 2019), IEEE, 8 April 2019 (2019-04-08), pages 1479-1482, XP033576708, DOI: 10.1109/ISBI.2019.8759580 [retrieved on 2019-07-10] | 1,3,4, 13-15 | INV. G06T7/00 G06T7/11 |
| Y | * abstract * | 2,9-11 | |
| A | * section 2 * | 5-8,12 | |
| | ----- | | |
| Y | VÁZQUEZ ANDREA ET AL: "FFClust: Fast fiber clustering for large tractography datasets for a detailed study of brain connectivity", NEUROIMAGE, ELSEVIER, AMSTERDAM, NL, vol. 220, 26 June 2020 (2020-06-26), XP086288685, ISSN: 1053-8119, DOI: 10.1016/J.NEUROIMAGE.2020.117070 [retrieved on 2020-06-26] * abstract * * figure 1 * * section 2.2 * | 2,9-11 | |
| | ----- | | **TECHNICAL FIELDS SEARCHED (IPC)** |
| A | GUEVARA P ET AL: "Automatic fiber bundle segmentation in massive tractography datasets using a multi-subject bundle atlas", NEUROIMAGE, ELSEVIER, AMSTERDAM, NL, vol. 61, no. 4, 24 February 2012 (2012-02-24), pages 1083-1099, XP028490439, ISSN: 1053-8119, DOI: 10.1016/J.NEUROIMAGE.2012.02.071 [retrieved on 2012-03-05] * the whole document * | 1-15 | G06T |
| | ----- | | |

The present search report has been drawn up for all claims

| Place of search | Date of completion of the search | Examiner |
|---|---|---|
| Munich | 6 January 2025 | Eveno, Nicolas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................

& : member of the same patent family, corresponding
document

EPO FORM 1503 03.82 (P04C01)